# EUROPEAN PATENT SPECIFICATION

(11) **EP 1 369 112 B1**
(45) Date of publication and mention of the grant of the patent: **29.11.2006**
(21) Application number: 03012812.8
(22) Date of filing: 05.06.2003
(51) Int. Cl.: A61K 9/70, A61K 47/32

(54) **Percutaneous absorption-type pharmaceutical preparation and process for producing the same**
Pharmazeutische Zusammensetzung zur Perkutann Absorption und Verfahren für ihre Herstellung
Préparation pharmaceutique pour absorption percutanée et procédé de fabrication

(30) Priority: 05.06.2002 JP 2002164973
(43) Date of publication of application: 10.12.2003
(73) Proprietor: NITTO DENKO CORPORATION, Osaka (JP)
(72) Inventor: Muraoka, Takateru, Ibaraki-shi, Osaka (JP); Inosaka, Keigo, Ibaraki-shi, Osaka (JP); Ishitani, Hiroko, Ibaraki-shi, Osaka (JP); Nakano, Yoshihisa, Ibaraki-shi, Osaka (JP)
(74) Representative: Grünecker, Kinkeldey, Stockmair & Schwanhäusser Anwaltssozietät

(56) References cited:
- EP-A- 0 531 938
- EP-A- 1 188 436
- US-A- 4 585 452
- US-A- 5 186 938
- US-A- 5 393 529

## Description

### FIELD OF THE INVENTION

The present invention relates to a percutaneous absorption-type pharmaceutical preparation for percutaneously administering a basic drug and a process for producing the same.

### BACKGROUND OF THE INVENTION

Various patch type pharmaceutical preparations including poultices and tape preparations are recently being developed as percutaneous absorption-type pharmaceutical preparations for administering a drug to the living body through the skin. Of these preparations, tape preparations containing a drug which exerts a systemic pharmacological action are especially attracting attention. For example, percutaneous absorption-type pharmaceutical preparations in a tape form which contain any of nitroglycerin, isosorbide dinitrate, various steroidal drugs, non-steroidal drugs, anesthetics, antihypertensive agents, and the like as an active ingredient in the pressure-sensitive adhesive layer were proposed, and some of them have come into the market. These percutaneous absorption-type pharmaceutical preparations employ an acrylic or synthetic-rubber-based pressure-sensitive adhesive containing any of various percutaneously absorbable drugs. Upon mere application to the skin, the drug is constantly absorbed into the body through the skin to show an excellent pharmacological action.

US 4,585,452 discloses a sustained release form suitable for transdermal administration of a drug comprising a first adhesive layer based upon an acrylic polymer and having dispersed therein a drug.

US 5,186,938 discloses an adhesive bilayer transdermal dosage system comprising a first component layer formed of a nitroglycrin-containing sheet of an at least partially crosslinked acrylic pressure-sensitive adhesive and a second component layer resistant to the passage of the drug.

Percutaneous absorption-type pharmaceutical preparations for the continuous percutaneous administration of drugs for treatments for or prevention of various diseases are desired to have sufficient adhesion to the skin and give an excellent wear feeling. In addition, the preparations are desired to have such a property that stripping thereof from the skin after wear does not result in the trouble in which the adhesive partly remains on the skin surface, i.e., the so-called adhesive remaining. However, percutaneous absorption-type pharmaceutical preparations heretofore in use, in particular, percutaneous absorption-type pharmaceutical preparations for the percutaneous absorption of basic drugs, have had a problem that properties of the pressure-sensitive adhesive change during wear and the pressure-sensitive adhesive layer tends to show a cohesive failure upon stripping, resulting in adhesive remaining.

On the other hand, a percutaneous absorption-type pharmaceutical preparation, after application to the skin, blocks up sweat glands in the skin and, as a result, perspiration occurs to cause, e.g., a phenomenon in which sweat resides between the skin and the percutaneous absorption-type pharmaceutical preparation. The degree of this perspiration varies considerably depending on the seasons. The sweat of the human being is mostly accounted for by water, and excessive perspiration causes the percutaneous absorption-type pharmaceutical preparation to peel off the skin or exerts other influences. The sweat contains various components besides water, such as lactic acid, urea, ammonia, and inorganic salts.

However, it has hitherto been thought that the influences of perspiration are within the range of fluctuations attributable to differences among individuals, seasonal differences, etc., and investigations for diminishing the influences of perspiration have been directed only toward minor modifications such as property improvements in pressure-sensitive adhesives and addition of additives or other ingredients. No sufficient investigation has been made on the stability of a percutaneous absorption-type pharmaceutical preparation in relation to sweat components, especially sweat components other than water.

### SUMMARY OF THE INVENTION

An object of the invention is to provide a stable percutaneous absorption-type pharmaceutical preparation for the percutaneous absorption of basic drugs which does not suffer a decrease in the cohesive force of the pressure-sensitive adhesive layer even in the presence of sweat components due to perspiration during wear and which is free from a cohesive failure and resultant adhesive remaining when stripped off. Another object of the invention is to provide a process for producing the pharmaceutical preparation.

The present inventors made intensive investigations in order to accomplish those objects. As a result, they have found that in percutaneous absorption-type pharmaceutical preparations containing a basic drug, the lactic acid contained in sweat is taken up by the pressure-sensitive adhesive layer under the influence of the basic drug and this lactic acid acts on crosslinks in the pressure-sensitive adhesive, which have been formed with a specific crosslinking agent, i.e., an organometallic compound, metal alcoholate, or metal chelate compound, to reduce the cohesive force of the pressure-sensitive adhesive layer. When this pharmaceutical preparation is stripped off, the reduced cohesive force of the pressure-sensitive adhesive layer results in a tendency to cohesive failure and hence causes the phenomenon of adhesive remaining. On the other hand, it has been found that when a crosslinking agent which forms crosslinks unsusceptible to the influence of lactic acid (e.g., a polyisocyanate compound) is used for crosslinking a pressure-sensitive adhesive containing a basic drug, then the formation of crosslinks is inhibited by the basic drug contained in the adhesive.

The inventors have further found that when a pressure-sensitive adhesive layer comprising a basic drug and either a pressure-sensitive adhesive crosslinked with a crosslinking agent which is not inhibited from forming crosslinks by the presence of the basic drug, e.g., a crosslinking agent such as an organometallic compound, metal alcoholate, or metal chelate compound, or an uncrosslinked pressure-sensitive adhesive is formed on one side of a substrate and a pressure-sensitive adhesive layer comprising a pressure-sensitive adhesive crosslinked with a crosslinking agent which forms crosslinks unsusceptible to the influence of lactic acid, i.e., a crosslinking agent which is other than an organometallic compound, metal alcoholate, and metal chelate compound, is formed on that pressure-sensitive adhesive layer, i.e., on the side to be applied to the skin, then a stable pharmaceutical preparation can be obtained which does not cause a decrease in the cohesive force of the pressure-sensitive adhesive layers even when the lactic acid in sweat is taken up and which is free from adhesive remaining when stripped off. Thus, the present invention has been completed.

The invention provides the following.
[1] A percutaneous absorption-type pharmaceutical preparation which comprises a substrate and, superposed on one side thereof in this order, a pressure-sensitive adhesive layer (A) comprising a pressure-sensitive adhesive crosslinked by physical crosslinking treatments with ultraviolet irradiation or electron beam irradiation, or crosslinked with a crosslinking agent selected from the group consisting of an organometallic compound a metal alcoholate or a metal chelate compound, and a basic drug, and a pressure-sensitive adhesive layer (B) comprising a pressure-sensitive adhesive crosslinked with a crosslinking agent which is other than an organometallic compound, metal alcoholate, and metal chelate compound.
[2] The percutaneous absorption-type pharmaceutical preparation described in [1] above wherein the pressure-sensitive adhesive of the pressure-sensitive adhesive layer (B) is a pressure-sensitive adhesive crosslinked with one or more crosslinking agents selected from the group consisting of polyisocyanate compounds, organic peroxides, melamine derivatives, polyfunctional compounds, amino resins, silane compounds, diol compounds, polyol compounds, bisphenol compounds, and sulfides.
[3] The percutaneous absorption-type pharmaceutical preparation described in [1] or [2] above wherein at least one of the pressure-sensitive adhesive layer (A) and the pressure-sensitive adhesive layer (B) contain a liquid plasticizing ingredient.
[4] The percutaneous absorption-type pharmaceutical preparation described in any one of [1] to [3] above wherein the pressure-sensitive adhesive of the pressure-sensitive adhesive layer (A) and the pressure-sensitive adhesive of the pressure-sensitive adhesive layer (B) have the same composition.
[5] The percutaneous absorption-type pharmaceutical preparation described in any one of [1] to [4] above wherein at least one of the pressure-sensitive adhesive of the pressure-sensitive adhesive layer (A) and the pressure-sensitive adhesive of the pressure-sensitive adhesive layer (B) each are an acrylic copolymer pressure-sensitive adhesive.
[6] The percutaneous absorption-type pharmaceutical preparation described in [5] above wherein the acrylic copolymer pressure-sensitive adhesive of each of the pressure-sensitive adhesive layer (A) and the pressure-sensitive adhesive layer (B) comprises a copolymer obtained by copolymerizing from 60 to 98% by weight of at least one alkyl (meth)acrylate in which the alkyl has 4 to 12 carbon atoms with from 2 to 40% by weight of at least one functional monomer.
[7] The percutaneous absorption-type pharmaceutical preparation described in [6] above wherein the functional monomer is a monomer having one or more substituents selected from the group consisting of a carboxyl group, a hydroxyl group, a sulfo group, an amino group, an amido group, an alkoxyl group, a cyano group, and an acyloxy group.
[8] The percutaneous absorption-type pharmaceutical preparation described in [7] above wherein the functional monomer is one or more monomers selected from the group consisting of (meth)acrylic acid, 2-hydroxyethyl (meth)acrylate, styrenesulfonic acid, (meth)acrylamide, vinylpyrrolidone, 2-aminoethyl (meth)acrylate, acrylonitrile, 2-methoxyethyl (meth)acrylate, and vinyl acetate.
[9] A process for producing a pharmaceutical preparation of the percutaneous absorption-type which comprises:
   (1) a step of forming a pressure-sensitive adhesive layer (A) comprising a pressure-sensitive adhesive and a basic drug on one side of a substrate; and
   (2) a step of crosslinking a pressure-sensitive adhesive with a crosslinking agent which is other than an organometallic compound, metal alcoholate, and metal chelate compound in the absence of any basic drug to obtain a crosslinked pressure-sensitive adhesive and forming a pressure-sensitive adhesive layer (B) comprising the crosslinked pressure-sensitive adhesive on the pressure-sensitive adhesive layer (A).

### DETAILED DESCRIPTION OF THE INVENTION

The invention will be explained below in detail.

The substrate to be used in the percutaneous absorption-type pharmaceutical preparation of the invention is not particularly limited. However, it is preferably made of a material which prevents the drug and other additives (e.g., a plasticizer and an absorption accelerator), incorporated in the pressure-sensitive adhesive layers from passing through the substrate and going out from the back side to result in a decrease in content. Namely, the substrate is preferably made of a material impermeable to these ingredients.

Examples of the substrate include films of single materials, such as films of polyesters (e.g., poly(ethylene terephthalate)), polyamides (e.g., nylons), polyolefins (e.g., polyethylene and polypropylene), poly(vinyl chloride), plasticized poly(vinyl chloride), plasticized vinyl acetate/vinyl chloride copolymers, poly(vinylidene chloride), ethylene/vinyl acetate copolymers, cellulose acetate, ethyl cellulose, ethylene/ethyl acrylate copolymers, polytetrafluoroethylene, polyurethanes, and ionomer resins and metal foils, e.g., aluminum foils. Examples thereof further include laminated films comprising a combination of two or more of these films.

The thickness of the substrate is not particularly limited. However, from the standpoint of not impairing soft feeling of the percutaneous absorption-type pharmaceutical preparation, the substrate thickness is generally from 1 to 25 µm, preferably from 1 to 15 µm.

The substrate preferably has a porous film laminated thereto so as to improve the anchoring (adhesion) of the pressure-sensitive adhesive layer to the substrate. In this case, the pressure-sensitive adhesive layers are formed on the porous-film side.

Examples of this porous film include papers, woven fabrics, nonwoven fabrics, and mechanically perforated films.

The pressure-sensitive adhesive to be used in the pressure-sensitive adhesive layer (A) is not particularly limited as long as it has pressure-sensitive adhesive properties at ordinary temperature. However, acrylic copolymer pressure-sensitive adhesives are preferred from the standpoints of adhesion to the skin, drug solubility, drug stability, etc. A single pressure-sensitive adhesive or a combination of two or more pressure-sensitive adhesives may be used. The acrylic copolymer pressure-sensitive adhesives are not particularly limited, and examples thereof include copolymers of at least one alkyl (meth)acrylate with at least one functional monomer. The term "functional monomer" as used herein means a monomer having at least one unsaturated double bond in the molecule and further having a functional group as or in a side chain. The copolymers of at least one alkyl (meth)acrylate with at least one functional monomer preferably are copolymers obtained by copolymerizing from 60 to 98% by weight, preferably from 65 to 97% by weight, of at least one alkyl (meth)acrylate with from 2 to 40% by weight, preferably from 3 to 35% by weight, of at least one functional monomer (provided that the sum of the monomers is 100% by weight).

Examples of the alkyl (meth)acrylate include the esters obtained from acrylic or methacrylic acid and linear or branched, primary, secondary, or tertiary alcohols in which the alkyl group has 4 to 12 carbon atoms.

Specific examples of the alkyl (meth)acrylate include butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, and 2-ethylhexyl (meth)acryiate.

Examples of the functional monomer include functional monomers having at least one unsaturated double bond in the molecule and further having one or more functional groups selected, for example, from the group consisting of carboxyl, hydroxyl, sulfo, amino, amido, alkoxyl, cyano, and acyloxy groups as or in a side chain. Specific examples of the functional monomer include alkoxyl-modified alkyl (meth)acrylate monomers obtained by modifying the alkyl group of an alkyl (meth)acrylate with a linear or branched alkoxyl group having 1 to 4 carbon atoms (e.g., methoxy or ethoxy) (such as, e.g., 2-methoxyethyl (meth)acrylate and 2-ethoxyethyl (meth)acrylate), acrylonitrile, vinyl acetate, vinyl propionate, vinylpyrrolidones (e.g., N-vinyl-2-pyrrolidone), vinylcaprolactam, (meth)acrylic acid, 2-hydroxyethyl (meth)acrylate, styrenesulfonic acid, (meth)acrylamide, and 2-aminoethyl (meth)acrylate.

Those alkyl (meth)acrylates may be used alone or in combination of two or more thereof, and those functional monomers may be used alone or in combination of two or more thereof.

Examples of the acrylic copolymer pressure-sensitive adhesives include copolymers of 2-ethylhexyl acrylate and acrylic acid, copolymers of 2-ethylhexyl acrylate, N-vinyl-2-pyrrolidone, and acrylic acid, and copolymers of 2-ethylhexyl acrylate and 2-hydroxyethyl acrylate.

A liquid plasticizing ingredient may be incorporated into the pressure-sensitive adhesive layer (A).

The liquid plasticizing ingredient is not particularly limited as long as it is liquid at ordinary temperature and compatible with the pressure-sensitive adhesive to be used (e.g., an acrylic copolymer pressure-sensitive adhesive).

When a liquid plasticizing ingredient compatible with the pressure-sensitive adhesive (acrylic copolymer pressure-sensitive adhesive) is incorporated into the pressure-sensitive adhesive layer and blends with the pressure-sensitive adhesive (acrylic copolymer pressure-sensitive adhesive) to form a stable homogeneous mixture, then it functions to plasticize the pressure-sensitive adhesive layer. The liquid plasticizing ingredient can be incorporated also for the purpose of further enhancing drug solubility in the pressure-sensitive adhesive.

The amount of the liquid plasticizing ingredient to be incorporated is generally from 10 to 200 parts by weight, preferably from 25 to 150 parts by weight, per 100 parts by weight of the pressure-sensitive adhesive. When the amount of the liquid plasticizing ingredient incorporated is 10 parts by weight or larger, preferably 25 parts by weight or larger, per 100 parts by weight of the pressure-sensitive adhesive, sufficient effects are assured with respect to plasticization, drug solubility, etc. When the amount of the liquid plasticizing ingredient incorporated is 200 parts by weight or smaller, preferably 150 parts by weight or smaller, per 100 parts by weight of the pressure-sensitive adhesive, the pressure-sensitive adhesive layer can be prevented from having an excessively reduced cohesive force and, hence, from arousing troubles such as adhesive remaining on the skin surface after stripping.

Examples of the liquid plasticizing ingredient include esters of fatty acids having 12 to 16 carbon atoms, monoglycerides of fatty acids having 8 to 10 carbon atoms, esters of dibasic acids having 6 to 10 carbon atoms, and nonionic surfactants. Such liquid plasticizing ingredients can be used alone or in combination of two or more thereof.

Although the pressure-sensitive adhesive in the pressure-sensitive adhesive layer (A) may be an uncrosslinked pressure-sensitive adhesive, it is desirable to crosslink the adhesive by an appropriate crosslinking technique especially when a liquid plasticizing ingredient is incorporated. Crosslinking can impart a moderate cohesive force to the pressure-sensitive adhesive layer.

Crosslinking reactions generally include physical crosslinking by ultraviolet irradiation, electron beam irradiation, and the like and chemical crosslinking with crosslinking agents such as polyisocyanate compounds, organic peroxides, organometallic compounds, metal alcoholates, metal chelate compounds, and polyfunctional compounds. In the invention, however, the pressure-sensitive adhesive of the pressure-sensitive adhesive layer (A), which contains a basic drug, and the pressure-sensitive adhesive of the pressure-sensitive adhesive layer (B), which will be described later, differ in the method of crosslinking.

For the pressure-sensitive adhesive of the pressure-sensitive adhesive layer (A), which contains a basic drug, crosslinking agents reactive with the basic drug, such as, e.g., polyisocyanate compounds, cannot be used because the basic drug inhibits these crosslinking agents from forming crosslinks. It is therefore necessary that the pressure-sensitive adhesive of the pressure-sensitive adhesive layer (A) be crosslinked by a crosslinking treatment in which crosslink formation is not inhibited by the presence of the basic drug.

Consequently, for crosslinking the pressure-sensitive adhesive of the pressure-sensitive adhesive layer (A), use may, for example, be made of: crosslinking treatments with a crosslinking agent which is not inhibited from forming crosslinks by the basic drug, such as, e.g., an organometallic compound (examples of which include zinc acetate, and zinc ammonium glycinate), a metal alcoholate (examples of which include tetraethyl titanate, tetraisopropyl titanate, aluminum isopropylate, and aluminum butylate), or a metal chelate compound (examples of which include diisopropoxy bis(acetylacetone)titanate, tetraoctylene glycol titanate, aluminum isopropylate, (ethyl acetoacetate) aluminum diisopropylate, aluminum tris(ethyl acetoacetate), and aluminum tris(acetylacetonate)); physical crosslinking treatments with ultraviolet irradiation or electron beam irradiation; or the like. Such crosslinking techniques may be used alone or in combination of two or more thereof.

The drug to be contained in the pressure-sensitive adhesive layer (A) is not particularly limited as long as it is a basic drug capable of being percutaneously absorbed. Examples thereof include heterocyclic derivatives which are not in the form of a pharmacologically acceptable salt but in a free form and have within the drug molecule at least one member selected from carboxylic acid derivatives, amino acid derivatives, amine derivatives, amic acid derivatives, aromatic amine derivatives, and a nitrogen atom.

Specific examples of the drug to be contained in the pressure-sensitive adhesive layer (A) include metoprolol, propranolol, azelastine, diazepam, clonidine, bisoprolol, pindolol, ifenprodil, and metoclopramide.

The basic drug can be incorporated into the pressure-sensitive adhesive layer (A) in the form of a solution or dispersion.

The basic drug to be contained in the pressure-sensitive adhesive layer (A) may be either a systemic drug or a topical drug.

Examples of the systemic drug include corticosteroids, analgetic anti-inflammatory agents, hypnotic sedatives, tranquilizing agents, antihypertensives, hypotensive diuretics, antibiotics, anesthetics, antibacterials, antifungal agents, vitamins, coronary vasodilators, antihistaminics, antitussives, sexual hormones, antidepressants, cerebral vasodilators, antiemetics, antitumor agents, and biodrugs. Examples of the topical drug include topical anesthetics, dental antibiotics, bactericidal disinfectants, infection preventive/therapeutic agents, anti-inflammatory agents, and adrenal cortex hormones.

The content of the basic drug in the pressure-sensitive adhesive layer (A) is in the range of generally from 0.2 to 80% by weight, preferably from 1 to 60% by weight, based on the whole weight of the pressure-sensitive adhesive layer (A).

The pressure-sensitive adhesive to be used in the pressure-sensitive adhesive layer (B) is not particularly limited as long as it has pressure-sensitive adhesive properties at ordinary temperature. However, acrylic copolymer pressure-sensitive adhesives are preferred from the standpoints of adhesion to the skin, drug solubility, drug stability, and reactivity in crosslinking. A single pressure-sensitive adhesive or a combination of two or more pressure-sensitive adhesives may be used. The acrylic copolymer pressure-sensitive adhesives for use in the pressure-sensitive adhesive layer (B) are not particularly limited, and examples thereof include copolymers of at least one alkyl (meth)acrylate with at least one functional monomer. The copolymers of at least one alkyl (meth)acrylate with at least one functional monomer preferably are copolymers obtained by copolymerizing from 60 to 98% by weight, preferably from 65 to 97% by weight, of at least one alkyl (meth)acrylate with from 2 to 40% by weight, preferably from 3 to 35% by weight, of at least one functional monomer (provided that the sum of the monomers is 100% by weight).

Examples of the alkyl (meth)acrylate include the esters obtained from acrylic or methacrylic acid and linear or branched, primary, secondary, or tertiary alcohols in which the alkyl group has 4 to 12 carbon atoms.

Specific examples of the alkyl (meth)acrylate include the same alkyl (meth)acrylates as those enumerated hereinabove with regard to the pressure-sensitive adhesive layer (A).

Examples of the functional monomer include functional monomers having at least one unsaturated double bond in the molecule and further having one or more functional groups selected, for example, from the group consisting of carboxyl, hydroxyl, sulfo, amino, amido, alkoxyl, cyano, and acyloxy groups as or in a side chain. Specific examples of the functional monomer include alkoxyl-modified alkyl (meth)acrylate monomers obtained by modifying the alkyl group of an alkyl (meth)acrylate with a linear or branched alkoxyl group having 1 to 4 carbon atoms (e.g., methoxy or ethoxy) (such as, e.g., 2-methoxyethyl (meth)acrylate and 2-ethoxyethyl (meth)acrylate), acrylonitrile, vinyl acetate, vinyl propionate, vinylpyrrolidones (e.g., N-vinyl-2-pyrrolidone), vinylcaprolactam, (meth)acrylic acid, 2-hydroxyethyl (meth)acrylate, styrenesulfonic acid, (meth)acrylamide, and 2-aminoethyl (meth)acrylate.

Those alkyl (meth)acrylates may be used alone or in combination of two or more thereof, and those functional monomers may be used alone or in combination of two or more thereof.

Examples of the acrylic copolymer pressure-sensitive adhesives include the same acrylic pressure-sensitive adhesives as those enumerated hereinabove with regard to the pressure-sensitive adhesive layer (A).

For crosslinking the pressure-sensitive adhesive of the pressure-sensitive adhesive layer (B), a crosslinking agent which is other than an organometallic compound, metal alcoholate, and metal chelate compound may be used. In other words, the pressure-sensitive adhesive of the pressure-sensitive adhesive layer (B) is substantially devoid of an organometallic compound, metal alcoholate, and metal chelate compound. Examples of the crosslinking agent other than an organometallic compound, metal alcoholate, and metal chelate compound include polyisocyanate compounds, organic peroxides, melamine derivatives, polyfunctional compounds, amino resins, silane compounds, diol compounds, polyol compounds, bisphenol compounds, and sulfides. These crosslinking agents may be used alone or in combination of two or more thereof.

The pressure-sensitive adhesive layer (B) contains no basic drug just after the production thereof. However, by superposing the pressure-sensitive adhesive layer (A), which contains a basic drug, on the pressure-sensitive adhesive layer (B) thereafter, a concentration gradient is formed and the drug moves into the superposed layers due to the concentration gradient. Usually, the pharmaceutical preparation comes to have a uniform drug concentration. As a result, due to the influence of the basic drug which has moved into the pressure-sensitive adhesive layer (B), the lactic acid contained in the sweat resulting from perspiration during wear is taken up by the pressure-sensitive adhesive layer. In case where the pressure-sensitive adhesive in the pressure-sensitive adhesive layer (B) has been crosslinked with an organometallic compound, metal alcoholate, or metal chelate compound as a crosslinking agent, the lactic acid taken up by the pressure-sensitive adhesive layer acts on crosslinks of the pressure-sensitive adhesive to reduce the cohesive force of the pressure-sensitive adhesive layer and thereby cause a cohesive failure when the pharmaceutical preparation is stripped off. Because of this, the pressure-sensitive adhesive of the pressure-sensitive adhesive layer (B), which is located on the side to be applied to the skin, is crosslinked with a crosslinking agent which is other than an organometallic compound, metal alcoholate, and metal chelate compound. Thus, a stable pharmaceutical preparation can be obtained in which the pressure-sensitive adhesive layer does not cause a decrease in cohesive force even when the lactic acid contained in sweat is taken up thereby, and which is hence free from a cohesive failure and resultant adhesive remaining when stripped off.

The amount of the crosslinking agent to be added varies depending on the kinds of the crosslinking agent and pressure-sensitive adhesive. However, the amount thereof is generally in the range of from 0.01 to 2 parts by weight, preferably from 0.03 to 1.5 parts by weight, per 100 parts by weight of the pressure-sensitive adhesive to be crosslinked.

A liquid plasticizing ingredient may be contained in the pressure-sensitive adhesive layer (B). The liquid plasticizing ingredient is not particularly limited as long as it is liquid at ordinary temperature and compatible with the pressure-sensitive adhesive to be used (e.g., an acrylic copolymer pressure-sensitive adhesive).

The amount of the liquid plasticizing ingredient to be incorporated in the pressure-sensitive adhesive layer (B) is generally from 10 to 200 parts by weight, preferably from 25 to 150 parts by weight, per 100 parts by weight of the pressure-sensitive adhesive. When the amount of the liquid plasticizing ingredient incorporated is 10 parts by weight or larger, preferably 25 parts by weight or larger, per 100 parts by weight of the pressure-sensitive adhesive, sufficient effects are obtained with respect to plasticization, drug solubility, etc. When the amount of the liquid plasticizing ingredient incorporated is 200 parts by weight or smaller, preferably 150 parts by weight or smaller, per 100 parts by weight of the pressure-sensitive adhesive, the pressure-sensitive adhesive layer can be prevented from having an excessively reduced cohesive force and, hence, from arousing troubles such as adhesive remaining on the skin surface after stripping.

From the standpoints of preventing delamination at the interface between the pressure-sensitive adhesive layers (A) and (B) after the bonding of the two pressure-sensitive adhesive layers, accelerating the movement of the drug from one to the other pressure-sensitive adhesive layer, and improving adhesion between the two pressure-sensitive adhesive layers, it is preferred that the pressure-sensitive adhesive of the pressure-sensitive adhesive layer (A) and the pressure-sensitive adhesive of the pressure-sensitive adhesive layer (B) should have the same composition. The term "same composition" implies that the pressure-sensitive adhesives are of the same kind or that when two or more kinds of pressure-sensitive adhesives are used, the two layers are equal in the kinds of pressure-sensitive adhesives and in the proportions thereof.

The thicknesses of the pressure-sensitive adhesive layer (A) and pressure-sensitive adhesive layer (B) are such that the total thickness of the pressure-sensitive adhesive layer (A) and the pressure-sensitive adhesive layer (B) superposed thereon is generally from 20 to 200 µm, preferably from 40 to 150 µm, from the standpoints of applicability to the skin and strippability. Although the pressure-sensitive adhesive layer (A) and pressure-sensitive adhesive layer (B) each may have any desired thickness, the ratio of the thickness of the pressure-sensitive adhesive layer (A) to that of the pressure-sensitive adhesive layer (B) is generally from 1:1 to 20:1, preferably from 2:1 to 15:1.

Additives may be incorporated into each of the pressure-sensitive adhesive layer (A) and pressure-sensitive adhesive layer (B) according to need. Examples thereof include antioxidants, various pigments, various fillers, stabilizers, drug dissolution aids, and drug dissolution inhibitors.

The percutaneous absorption-type pharmaceutical preparation of the invention can be produced, for example, by a process comprising the following steps (1) and (2).

Namely, the pharmaceutical preparation can be produced through:
step (1) of forming a pressure-sensitive adhesive layer (A) comprising a pressure-sensitive adhesive and a basic drug on one side of a substrate; and
step (2) of crosslinking a pressure-sensitive adhesive with a crosslinking agent which is other than an organometallic compound, metal alcoholate, and metal chelate compound in the absence of any basic drug to obtain a crosslinked pressure-sensitive adhesive and forming a pressure-sensitive adhesive layer (B) comprising the crosslinked pressure-sensitive adhesive on the pressure-sensitive adhesive layer (A).

In step (1), a pressure-sensitive adhesive layer (A) can be formed, for example, by a method which comprises dissolving or dispersing a pressure-sensitive adhesive (e.g., an acrylic copolymer pressure-sensitive adhesive) and a basic drug in a solvent or dispersion medium optionally together with a crosslinking agent, a liquid plasticizing ingredient, and other additives, applying the resultant solution or dispersion to one side of a substrate, and drying the coating to form the pressure-sensitive adhesive layer (A). An alternative method is that comprising applying the solution or dispersion to a separator (e.g., a polyester film treated with a releasant), drying the coating to form a pressure-sensitive adhesive layer, and then transferring the pressure-sensitive adhesive layer to one side of a substrate to form the pressure-sensitive adhesive layer (A) .

In step (2), a pressure-sensitive adhesive layer (B) can be formed, for example, in the following manner. A pressure-sensitive adhesive (e.g., an acrylic copolymer pressure-sensitive adhesive) and a crosslinking agent which is other than an organometallic compound, metal alcoholate, and metal chelate are dissolved or dispersed in a solvent or dispersion medium optionally together with a liquid plasticizing ingredient and other additives. The resultant solution or dispersion is applied to one side of a separator (e.g., a polyester film treated with a releasant) and the coating is dried to form a pressure-sensitive adhesive layer comprising a crosslinked pressure-sensitive adhesive. Thereafter, this pressure-sensitive adhesive layer is bonded to the pressure-sensitive adhesive layer (A) by a known method so that the pressure-sensitive adhesive layers come into direct contact with each other. Thus, the pressure-sensitive adhesive layer (B) can be formed. As the separator, the release sheet which will be described later may be used.

The solvent or dispersion medium to be used for forming the pressure-sensitive adhesive layer (A) is not particularly limited, and can be selected from solvents or dispersion media ordinary used for pressure-sensitive adhesives while taking into consideration the kind of the pressure-sensitive adhesive, reactivity with the drug, etc. Examples thereof include ethyl acetate, toluene, hexane, 2-propanol, methanol, and ethanol.

The solvent or dispersion medium to be used for forming the pressure-sensitive adhesive layer (B) is not particularly limited, and can be selected from solvents or dispersion media ordinary used for pressure-sensitive adhesives while taking into consideration the kind of the pressure-sensitive adhesive, reactivity with the crosslinking agent, etc. Examples thereof include ethyl acetate, toluene, hexane, 2-propanol, methanol, and ethanol.

The pharmaceutical preparation obtained through steps (1) and (2) is a layered product which, just after the production thereof, comprises a drug-containing pressure-sensitive adhesive layer (pressure-sensitive adhesive layer (A)) and a drug-free pressure-sensitive adhesive layer (pressure-sensitive adhesive layer(B)). However, in order for this layered product to be used as a pharmaceutical preparation, it is desirably made to be a stable pharmaceutical preparation finally having an even concentration. Drug movement from one to the other superposed layer may be accelerated by storing the layered product comprising the pressure-sensitive adhesive layer (A) and the pressure-sensitive adhesive layer (B), for example, at a certain constant temperature.

It is preferred in the percutaneous absorption-type pharmaceutical preparation of the invention that the exposed side of the pressure-sensitive adhesive layer (B) be covered and protected with a release sheet until just before application to the skin. The release sheet is stripped off to expose the pressure-sensitive adhesive layer surface just before use, and this pharmaceutical preparation is applied to the skin to administer the drug. The release sheet is not particularly limited as long as it can be easily stripped from the pressure-sensitive adhesive layer just before use. For example, use is made of a film of a polyester, poly(vinyl chloride), poly(vinylidene chloride), poly(ethylene terephthalate), or the like in which the side to be in contact with the pressure-sensitive adhesive layer has been treated with a silicone, or of a laminated film obtained by laminating a polyolefin to wood-free paper or glassine paper. The thickness of the release sheet is generally 1,000 µm or smaller, preferably from 30 to 200 µm.

The shape of the percutaneous absorption-type pharmaceutical preparation of the invention is not particularly limited. Examples thereof include tape forms and sheet forms.

The dose of the percutaneous absorption-type pharmaceutical preparation of the invention varies depending on the kind of the drug used, the age, body weight, and condition of the patient, etc. Usually, however, the dose for an adult is such that the pharmaceutical preparation containing from 1 to 500 mg of a percutaneously absorbable drug is applied to an area of from 1 to 100 cm² and about from once per day to once per 7 days.

The invention will be explained below in more detail by reference to Examples, Comparative Examples, and Experimental Examples, but the invention should not be construed as being limited by these in any way. In the following description, all parts and percents are by weight.

### Preparation of Acrylic Copolymer Pressure-Sensitive Adhesives:

In an inert gas atmosphere, 95 parts of 2-ethylhexyl acrylate was copolymerized with 5 parts of acrylic acid in ethyl acetate to prepare an acrylic copolymer pressure-sensitive adhesive (hereinafter referred to as "acrylic copolymer pressure-sensitive adhesive (a)").

In an inert gas atmosphere, 72 parts of 2-ethylhexyl acrylate was copolymerized with 25 parts of N-vinyl-2-pyrrolidone and 3 parts of acrylic acid in ethyl acetate to prepare an acrylic copolymer pressure-sensitive adhesive (hereinafter referred to as "acrylic copolymer pressure-sensitive adhesive (b)").

In an inert gas atmosphere, 60 parts of 2-ethylhexyl acrylate was copolymerized with 10 parts of 2-hydroxyethyl acrylate and 30 parts of vinyl acetate in ethyl acetate to prepare an acrylic copolymer pressure-sensitive adhesive (hereinafter referred to as "acrylic copolymer pressure-sensitive adhesive (c)").

### EXAMPLE 1

An ethyl acetate solution (pressure-sensitive adhesive solution for pressure-sensitive adhesive layer (A)) containing 46 parts of acrylic copolymer pressure-sensitive adhesive (a), 4 parts of metoprolol, 50 parts of isopropyl myristate (IPM), and 0.3 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 40 µm on a dry basis. The coating was dried to form a pressure-sensitive adhesive layer (A).

An ethyl acetate solution (pressure-sensitive adhesive solution for pressure-sensitive adhesive layer (B)) containing 47.9 parts of acrylic copolymer pressure-sensitive adhesive (a), 52.1 part of IPM, and 0.2 parts of a polyisocyanate (Coronate HL (C/HL), manufactured by Nippon Polyurethane Co., Ltd.) was applied to a release sheet made of a polyester (75 µm thick) in such an amount as to result in a thickness of 40 µm on a dry basis. The coating was dried to form a pressure-sensitive adhesive layer (B).

Subsequently, the pressure-sensitive adhesive layer (A) was bonded to the pressure-sensitive adhesive layer (B) so that these adhesive layers came into direct contact with each other. Thus, a percutaneous absorption-type pharmaceutical preparation was produced. After the production, this pharmaceutical preparation was heated at 70°C for 48 hours for the purposes of accelerating crosslinking and accelerating drug movement from one to the other layer.

### EXAMPLE 2

A percutaneous absorption-type pharmaceutical preparation was produced in the same manner as in Example 1, except that (ethyl acetoacetate)aluminum diisopropylate was not incorporated into the pressure-sensitive adhesive solution for pressure-sensitive adhesive layer (A). After the production, this pharmaceutical preparation was heated at 70°C for 48 hours as in Example 1.

### EXAMPLE 3

A percutaneous absorption-type pharmaceutical preparation was produced in the same manner as in Example 1, except that the pressure-sensitive adhesive solutions were applied in such respective amounts as to give a pressure-sensitive adhesive layer (A) having a thickness of 60 µm on a dry basis and a pressure-sensitive adhesive layer (B) having a thickness of 20 µm on a dry basis. After the production, the pharmaceutical preparation was heated at 70°C for 48 hours as in Example 1.

### EXAMPLE 4

An ethyl acetate solution (pressure-sensitive adhesive solution for pressure-sensitive adhesive layer (A)) containing 45 parts of acrylic copolymer pressure-sensitive adhesive (b), 10 parts of propranolol, 45 parts of IPM, and 0.3 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 40 µm on a dry basis. The coating was dried to form a pressure-sensitive adhesive layer (A).

An ethyl acetate solution (pressure-sensitive adhesive solution for pressure-sensitive adhesive layer (B)) containing 50 parts of acrylic copolymer pressure-sensitive adhesive (b), 50 parts of IPM, and 0.3 parts of a polyisocyanate (C/HL, manufactured by Nippon Polyurethane Co., Ltd.) was applied to a release sheet made of a polyester (75 µm thick) in such an amount as to result in a thickness of 40 µm on a dry basis. The coating was dried to form a pressure-sensitive adhesive layer (B).

Subsequently, the pressure-sensitive adhesive layer (A) was bonded to the pressure-sensitive adhesive layer (B) so that these adhesive layers came into direct contact with each other. Thus, a percutaneous absorption-type pharmaceutical preparation was produced. After the production, this pharmaceutical preparation was heated at 70°C for 48 hours for the purposes of accelerating crosslinking and accelerating drug movement from one to the other layer.

### EXAMPLE 5

A percutaneous absorption-type pharmaceutical preparation was produced in the same manner as in Example 4, except that (ethyl acetoacetate)aluminum diisopropylate was not incorporated into the pressure-sensitive adhesive solution for pressure-sensitive adhesive layer (A). After the production, this pharmaceutical preparation was heated at 70°C for 48 hours as in Example 4.

### EXAMPLE 6

An ethyl acetate solution (pressure-sensitive adhesive solution for pressure-sensitive adhesive layer (A)) containing 50 parts of acrylic copolymer pressure-sensitive adhesive (c), 10 parts of azelastine, 40 parts of IPM, and 0.3 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 60 µm on a dry basis. The coating was dried to form a pressure-sensitive adhesive layer (A).

An ethyl acetate solution (pressure-sensitive adhesive solution for pressure-sensitive adhesive layer (B)) containing 55.6 parts of acrylic copolymer pressure-sensitive adhesive (c), 44.4 parts of IPM, and 0.3 parts of a polyisocyanate (C/HL, manufactured by Nippon Polyurethane Co., Ltd.) was applied to a release sheet made of a polyester (75 µm thick) in such an amount as to result in a thickness of 20 µm on a dry basis. The coating was dried to form a pressure-sensitive adhesive layer (B).

Subsequently, the pressure-sensitive adhesive layer (A) was bonded to the pressure-sensitive adhesive layer (B) so that these adhesive layers came into direct contact with each other. Thus, a percutaneous absorption-type pharmaceutical preparation was produced. After the production, this pharmaceutical preparation was heated at 70°C for 48 hours for the purposes of accelerating crosslinking and accelerating drug movement from one to the other layer.

### EXAMPLE 7

A percutaneous absorption-type pharmaceutical preparation was produced in the same manner as in Example 6, except that (ethyl acetoacetate)aluminum diisopropylate was not incorporated into the pressure-sensitive adhesive solution for pressure-sensitive adhesive layer (A). After the production, this pharmaceutical preparation was heated at 70°C for 48 hours as in Example 6.

### COMPARATIVE EXAMPLE 1

An ethyl acetate solution containing 46 parts of acrylic copolymer pressure-sensitive adhesive (a), 4 parts of metoprolol, 50 parts of IPM, and 0.3 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 80 µm on a dry basis. The coating was dried to produce a percutaneous absorption-type pharmaceutical preparation. After the production, this pharmaceutical preparation was heated at 70°C for 48 hours.

### COMPARATIVE EXAMPLE 2

An ethyl acetate solution containing 46 parts of acrylic copolymer pressure-sensitive adhesive (a), 4 parts of metoprolol, and 50 parts of IPM was applied to the nonwoven-fabric side of a laminated film composed of a nonwoven polyester fabric (basis weight, 12 g/m²) and a polyester film (2 µm thick) in such an amount as to result in a thickness of 40 µm on a dry basis. The coating was dried to form a drug-containing pressure-sensitive adhesive layer.

An ethyl acetate solution containing 47.9 parts of acrylic copolymer pressure-sensitive adhesive (a), 52.1 part of IPM, and 0.3 parts of (ethyl acetoacetate)aluminum diisopropylate was applied to a release sheet made of a polyester (75 µm thick) in such an amount as to result in a thickness of 40 µm on a dry basis. The coating was dried to form a drug-free pressure-sensitive adhesive layer.

Subsequently, the drug-containing pressure-sensitive adhesive layer was bonded to the drug-free pressure-sensitive adhesive layer so that these adhesive layers came into direct contact with each other. Thus, a percutaneous absorption-type pharmaceutical preparation was produced. After the production, this pharmaceutical preparation was heated at 70°C for 48 hours.

### COMPARATIVE EXAMPLE 3

An ethyl acetate solution containing 45 parts of acrylic copolymer pressure-sensitive adhesive (b), 10 parts of propranolol, and 45 parts of IPM was applied to the nonwoven-fabric side of a laminated film composed of a nonwoven polyester fabric (basis weight, 12 g/m²) and a polyester film (2 µm thick) in such an amount as to result in a thickness of 40 µm on a dry basis. The coating was dried to form a drug-containing pressure-sensitive adhesive layer.

An ethyl acetate solution containing 50 parts of acrylic copolymer pressure-sensitive adhesive (b), 50 parts of IPM, and 0.3 parts of (ethyl acetoacetate)aluminum diisopropylate was applied to a release sheet made of a polyester (75 µm thick) in such an amount as to result in a thickness of 40 µm on a dry basis. The coating was dried to form a drug-free pressure-sensitive adhesive layer.

Subsequently, the drug-containing pressure-sensitive adhesive layer was bonded to the drug-free pressure-sensitive adhesive layer so that these adhesive layers came into direct contact with each other. Thus, a percutaneous absorption-type pharmaceutical preparation was produced. After the production, this pharmaceutical preparation was heated at 70°C for 48 hours.

### COMPARATIVE EXAMPLE 4

An ethyl acetate solution containing 50 parts of acrylic copolymer pressure-sensitive adhesive (c), 10 parts of azelastine, and 40 parts of IPM was applied to the nonwoven-fabric side of a laminated film composed of a nonwoven polyester fabric (basis weight, 12 g/m²) and a polyester film (2 µm thick) in such an amount as to result in a thickness of 60 µm on a dry basis. The coating was dried to form a drug-containing pressure-sensitive adhesive layer.

An ethyl acetate solution containing 55.6 parts of acrylic copolymer pressure-sensitive adhesive (c), 44.4 parts of IPM, and 0.4 parts of (ethyl acetoacetate)aluminum diisopropylate was applied to a release sheet made of a polyester (75 µm thick) in such an amount as to result in a thickness of 20 µm on a dry basis. The coating was dried to form a drug-free pressure-sensitive adhesive layer.

Subsequently, the drug-containing pressure-sensitive adhesive layer was bonded to the drug-free pressure-sensitive adhesive layer so that these adhesive layers came into direct contact with each other. Thus, a percutaneous absorption-type pharmaceutical preparation was produced. After the production, this pharmaceutical preparation was heated at 70°C for 48 hours.

### COMPARATIVE EXAMPLE 5

An ethyl acetate solution containing 45 parts of acrylic copolymer pressure-sensitive adhesive (a), 15 parts of isosorbide dinitrate, 40 parts of IPM, and 0.3 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 60 µm on a dry basis. The coating was dried to produce a percutaneous absorption-type pharmaceutical preparation. After the production, this pharmaceutical preparation was heated at 70°C for 48 hours.

### COMPARATIVE EXAMPLE 6

An ethyl acetate solution containing 47 parts of acrylic copolymer pressure-sensitive adhesive (b), 3 parts of estradiol, 50 parts of IPM, and 0.4 parts of (ethyl acetoacetate)aluminum diisopropylate was applied to the nonwoven-fabric side of a laminated film composed of a nonwoven polyester fabric (basis weight, 12 g/m²) and a polyester film (2 µm thick) in such an amount as to result in a thickness of 60 µm on a dry basis. The coating was dried to produce a percutaneous absorption-type pharmaceutical preparation. After the production, this pharmaceutical preparation was heated at 70°C for 48 hours.

### COMPARATIVE EXAMPLE 7

An ethyl acetate solution containing 47 parts of acrylic copolymer pressure-sensitive adhesive (b), 3 parts of estradiol, and 50 parts of IPM was applied to the nonwoven-fabric side of a laminated film composed of a nonwoven polyester fabric (basis weight, 12 g/m²) and a polyester film (2 µm thick) in such an amount as to result in a thickness of 40 µm on a dry basis. The coating was dried to form a drug-containing pressure-sensitive adhesive layer.

An ethyl acetate solution containing 48.5 parts of acrylic copolymer pressure-sensitive adhesive (b), 51.5 parts of IPM, and 0.4 parts of (ethyl acetoacetate)aluminum diisopropylate was applied to a release sheet made of a polyester (75 µm thick) in such an amount as to result in a thickness of 20 µm on a dry basis. The coating was dried to form a basic-drug-free pressure-sensitive adhesive layer.

Subsequently, the basic-drug-containing pressure-sensitive adhesive layer was bonded to the basic-drug-free pressure-sensitive adhesive layer so that these adhesive layers came into direct contact with each other. Thus, a percutaneous absorption-type pharmaceutical preparation was produced. After the production, this pharmaceutical preparation was heated at 70°C for 48 hours.

In Tables 1 and 2 are shown the compositions and dry thicknesses of the pressure-sensitive adhesive layers in each of Examples 1 to 7 and Comparative Examples 1 to 7.

**Table 1**

| | Pressure-Sensitive Adhesive Layer (A) | | | | | Pressure-Sensitive Adhesive Layer (B) | | | |
|---|---|---|---|---|---|---|---|---|---|
| Example No. | Pressure-sensitive adhesive (parts) | Percutaneously absorbable drug (parts) | Liquid plasticizing ingredient (parts) | Cross-linking agent (parts) | Dry thickness (µm) | Pressure-sensitive adhesive (parts) | Liquid plasticizing ingredient (parts) | Cross-linking agent (parts) | Dry thickness (µm) |
| Ex. 1 | adhesive (a) 46 | metoprolol 4 | IPM 50 | ALCH 0.3 | 40 | adhesive (a) 47.9 | IPM 52.1 | C/HL 0.2 | 40 |
| Ex. 2 | adhesive (a) 46 | metoprolol 4 | IPM 50 | - | 40 | adhesive (a) 47.9 | IPM 52.1 | C/HL 0.2 | 40 |
| Ex. 3 | adhesive (a) 46 | metoprolol 4 | IPM 50 | ALCH 0.3 | 60 | adhesive (a) 47.9 | IPM 52.1 | C/HL 0.2 | 20 |
| Ex. 4 | adhesive (b) 45 | propranol 10 | IPM 45 | ALCH 0.3 | 40 | adhesive (b) 50 | IPM 50 | C/HL 0.3 | 40 |
| Ex. 5 | adhesive (b) 45 | propranol 10 | IPM 45 | - | 40 | adhesive (b) 50 | IPM 50 | C/HL 0.3 | 40 |
| Ex. 6 | adhesive (c) 50 | azelastine 10 | IPM 40 | ALCH 0.3 | 60 | adhesive (c) 55.6 | IPM 44.4 | C/HL 0.3 | 20 |
| Ex. 7 | adhesive (c) 50 | azelastine 10 | IPM 40 | - | 60 | adhesive (c) 55.6 | IPM 44.4 | C/HL 0.3 | 20 |

| | | | | | | | | | |
|---|---|---|---|---|---|---|---|---|---|
| IPM: isopropyl myristate ALCH: (ethyl acetoacetate)aluminum diisopropylate C/HL: polyisocyanate | | | | | | | | | |

**Table 2**

| | Drug-Containing Pressure-Sensitive Adhesive Layer (A) | | | | | Drug-Free Pressure-Sensitive Adhesive Layer (B) | | | |
|---|---|---|---|---|---|---|---|---|---|
| Comparative Example No. | Pressure-sensitive adhesive (parts) | Percutaneously absorbable drug (parts) | Liquid plasticizing ingredient (parts) | Cross-linking agent (parts) | Dry thickness (µm) | Pressure-sensitive adhesive (parts) | Liquid plasticizing ingredient (parts) | Cross-linking agent (parts) | Dry thickness (µm) |
| Comp. Ex. 1 | adhesive (a) 46 | metoprolol 4 | IPM 50 | ALCH 0.3 | 80 | - | - | - | - |
| Comp. Ex. 2 | adhesive (a) 46 | metoprolol 4 | IPM 50 | - | 40 | adhesive (a) 47.9 | IPM 52.1 | ALCH 0.3 | 40 |
| Comp. Ex. 3 | adhesive (b) 45 | propranol 10 | IPM 45 | - | 40 | adhesive (b) 50 | IPM 50 | ALCH 0.3 | 40 |
| Comp. Ex. 4 | adhesive (c) 50 | azelastine 10 | IPM 40 | - | 60 | adhesive (c) 55.6 | IPM 44.4 | ALCH 0.4 | 20 |
| Comp. Ex. 5 | adhesive (a) 45 | isosorbide 15 | IPM 40 | ALCH 0.3 | 60 | - | - | - | - |
| Comp.- Ex. 6 | adhesive (b) 47 | estradiol 3 | IPM 50 | ALCH 0.4 | 60 | - | - | - | - |
| Comp. Ex. 7 | adhesive (b) 47 | estradiol 3 | IPM 50 | - | 40 | adhesive (b) 48.5 | IPM 51.5 | ALCH 0.4 | 20 |

| | | | | | | | | | |
|---|---|---|---|---|---|---|---|---|---|
| IPM: isopropyl myristate ALCH: (ethyl acetoacetate)aluminum diisopropylate C/HL: polyisocyana | | | | | | | | | |

### EXPERIMENTAL EXAMPLES

The percutaneous absorption-type pharmaceutical preparations produced in the Examples and Comparative Examples given above were subjected to the lactic acid uptake test and adhesive force measurement shown below. EXPERIMENTAL EXAMPLE 1 - Lactic Acid Uptake Test

The amount of lactic acid taken up by a pharmaceutical preparation was measured by the following method. In a petri dish was placed 15 mL of 1% aqueous lactic acid solution. A 30-cm² specimen punched out of the pharmaceutical preparation was immersed therein for 10 minutes and the excess lactic acid solution was then removed (lactic acid immersion treatment). This pharmaceutical preparation was chopped and immersed in 15 mL of distilled water placed in a meyer flask, and 5 mL of an internal standard solution was added thereto. This mixture was shaken at 40°C for 1 hour for extraction.
The resultant extract was examined by HPLC under the following conditions to determine the amount of lactic acid absorbed in the pharmaceutical preparation. The results obtained are shown in Table 3.

The HPLC conditions used are as follows.
(Conditions for Lactic Acid Determination)
Column: YMC-Pack PolymerC18 (φ4.6 x 250 mm)
Moving phase: 0.1% phosphoric acid
Column temperature: 25°C
Flow rate: 1.0 mL/min
Detection method: absorbance measurement at UV 210 nm Internal standard solution: aqueous acetic acid solution (0.5→1000)

### EXPERIMENTAL EXAMPLE 2 - Adhesive Force Measurement

The adhesive force of each percutaneous absorption-type pharmaceutical preparation produced (hereinafter referred to as "adhesive force 1") and the adhesive force of a sample obtained by subjecting each percutaneous absorption-type pharmaceutical preparation to a lactic acid immersion treatment under the same conditions as in the lactic acid uptake test described above, applying the treated pharmaceutical preparation to a release sheet, and then allowing it to stand for 24 hours (hereinafter referred to as "adhesive force 2") each were measured by the following method. The pharmaceutical preparation was cut into a strip having a width of 24 mm. The pressure-sensitive adhesive side of this strip of the pharmaceutical preparation was applied to a Bakelite plate and press-bonded thereto by rolling a 300-g roller forward and backward once thereon. Thereafter, the pharmaceutical preparation was peeled from the plate in the 180° direction at a rate of 300 mm/min and the adhesive force in this peeling was measured. The results obtained are shown in Table 3.

**Table 3**

| | Amount of lactic acid taken up (mg/cm²) | Adhesive forces (g/24 mm) | |
|---|---|---|---|
| | | Adhesive force 1 | Adhesive force 2 |
| Example 1 | 0.042 | 98 | 102 |
| Example 2 | 0.045 | 92 | 95 |
| Example 3 | 0.038 | 89 | 90 |
| Example 4 | 0.052 | 125 | 119 |
| Example 5 | 0.048 | 132 | 129 |
| Example 6 | 0.032 | 112 | 109 |
| Example 7 | 0.036 | 115 | 120 |
| Comparative Example 1 | 0.044 | 96 | 425 |
| Comparative Example 2 | 0.051 | 94 | 512 |
| Comparative Example 3 | 0.046 | 122 | 621 |
| Comparative Example 4 | 0.035 | 109 | 385 |
| Comparative Example 5 | 0.002 | 87 | 88 |
| Comparative Example 6 | 0.003 | 116 | 121 |
| Comparative Example 7 | 0.004 | 118 | 124 |

Table 3 shows the following. The basic-drug-containing percutaneous absorption-type pharmaceutical preparations of Examples 1 to 7 according to the invention each took up lactic acid but had almost no difference between adhesive force 1 and adhesive force 2. Namely, these were stable pharmaceutical preparations unsusceptible to the influence of lactic acid.

In contrast, the basic-drug-containing pharmaceutical preparations of Comparative Examples 1 to 4 each were an unstable preparation which took up lactic acid and suffered a considerable change in adhesive force due to the lactic acid.

The basic-drug-free pharmaceutical preparations of Comparative Examples 5 to 7 each showed no difference between adhesive force 1 and adhesive force 2. This indicates that in basic-drug-free pharmaceutical preparations, lactic acid is not taken up by the preparations depending on the kind of the drug.

The percutaneous absorption-type pharmaceutical preparation of the invention can be prevented from suffering a decrease in the cohesive force of the pressure-sensitive adhesive layer when lactic acid as a sweat component is taken up. Consequently, the present invention provides: a stable percutaneous absorption-type pharmaceutical preparation for the percutaneous absorption of basic drugs which does not cause a decrease in the cohesive force of the pressure-sensitive adhesive layer even in the presence of sweat components due to perspiration during wear and which is free from a cohesive failure and resultant adhesive remaining when stripped off; and a process for producing the pharmaceutical preparation.

While the invention has been described in detail and with reference to specific embodiments thereof, it will be apparent to one skilled in the art that various changes and modifications can be made therein without departing from the scope thereof.

This application is based on Japanese patent application No. 2002-164973 filed June 5, 2002.

## Claims

1. A percutaneous absorption-type pharmaceutical preparation which comprises a substrate and, superposed on one side thereof in this order,
a pressure-sensitive adhesive layer (A) comprising a pressure-sensitive adhesive crosslinked by physical crosslinking treatments with ultraviolet irradiation or electron beam irradiation, or crosslinked with a crosslinking agent selected from the group consisting of an organometallic compound, a metal alcoholate or a metal chelate compound, and a basic drug, and
a pressure-sensitive adhesive layer (B) comprising a pressure-sensitive adhesive crosslinked with a crosslinking agent other than an organometallic compound, metal alcoholate, and metal chelate compound.

2. The percutaneous absorption-type pharmaceutical preparation of claim 1, wherein the pressure-sensitive adhesive of the pressure-sensitive adhesive layer (B) is a pressure-sensitive adhesive crosslinked with one or more crosslinking agents selected from the group consisting of polyisocyanate compounds, organic peroxides, melamine derivatives, polyfunctional compounds, amino resins, silane compounds, diol compounds, polyol compounds, bisphenol compounds, and sulfides.

3. The percutaneous absorption-type pharmaceutical preparation of claim 1, wherein at least one of the pressure-sensitive adhesive layer (A) and the pressure-sensitive adhesive layer (B) contain a liquid plasticizing ingredient.

4. The percutaneous absorption-type pharmaceutical preparation of claim 1, wherein the pressure-sensitive adhesive of the pressure-sensitive adhesive layer (A) and the pressure-sensitive adhesive of the pressure-sensitive adhesive layer (B) have the same composition.

5. The percutaneous absorption-type pharmaceutical preparation of claim 1, wherein at least one of the pressure-sensitive adhesive of the pressure-sensitive adhesive layer (A) and the pressure-sensitive adhesive of the pressure-sensitive adhesive layer (B) each are an acrylic copolymer pressure-sensitive adhesive.

6. The percutaneous absorption-type pharmaceutical preparation of claim 5, wherein the acrylic copolymer pressure-sensitive adhesive of each of the pressure-sensitive adhesive layer (A) or the pressure-sensitive adhesive layer (B) comprises a copolymer obtained by copolymerizing from 60 to 98% by weight of at least one alkyl (meth)acrylate in which the alkyl has 4 to 12 carbon atoms with from 2 to 40% by weight of at least one functional monomer.

7. The percutaneous absorption-type pharmaceutical preparation of claim 6, wherein the functional monomer is a monomer having one or more substituents selected from the group consisting of a carboxyl group, a hydroxyl group, a sulfo group, an amino group, an amido group, an alkoxyl group, a cyano group, and an acyloxy group.

8. The percutaneous absorption-type pharmaceutical preparation of claim 7, wherein the functional monomer is one or more monomers selected from the group consisting of (meth)acrylic acid, 2-hydroxyethyl (meth)acrylate, styrenesulfonic acid, (meth)acrylamide, vinylpyrrolidone, 2-aminoethyl (meth)acrylate, acrylonitrile, 2-methoxyethyl (meth)acrylate, and vinyl acetate.

9. A process for producing a pharmaceutical preparation of the percutaneous absorption-type which comprises;
(1) a step of forming a pressure-sensitive adhesive layer (A) comprising a pressure-sensitive adhesive crosslinked by physical crosslinking treatments with ultraviolet irradiation or electron beam irradiation, or crosslinked with a crosslinking agent selected from the group consisting of an organometallic compound, a metal alcoholate or a metal chelate compound, and a basic drug on one side of a substrate; and
(2) a step of crosslinking a pressure-sensitive adhesive with a crosslinking agent other than an organometallic compound, metal alcoholate, and metal chelate compound in the absence of any basic drug to obtain a crosslinked pressure-sensitive adhesive and forming a pressure-sensitive adhesive layer (B) comprising the crosslinked pressure-sensitive adhesive on the pressure-sensitive adhesive layer (A).

## Patentansprüche

1. Eine perkutane pharmazeutische Zubereitung vom Absorptionstyp, umfassend ein Substrat und übereinanderliegend auf einer Seite desselben in dieser Reihenfolge,
eine Haftkleberschicht (A), umfassend einen Haftkleber, der durch physikalische Vernetzungsverfahren mit ultravioletter Strahlung oder Bestrahlung mit einem Elektronenstrahl vernetzt wurde, oder der mit einem Vernetzer, ausgewählt aus der Gruppe, bestehend aus einer metallorganischen Verbindung, einem Metallalkoholat oder einer Metallchelatverbindung vernetzt wurde, und ein basisches Arzneimittel, und
eine Haftkleberschicht (B), umfassend einen Haftkleber, der mit einem anderen Vernetzer als einer metallorganischen Verbindung, einem Metallalkoholat und einer Metallchelatverbindung vemetzt wurde.

2. Die perkutane pharmazeutische Zubereitung vom Absorptionstyp des Anspruchs 1, worin der Haftkleber der Haftkleberschicht (B) ein Haftkleber ist,
der mit einem oder mehreren Vemetzem, ausgewählt aus der Gruppe, bestehend aus Polyisocyanatverbindungen, organischen Peroxiden, Melaminderivaten, polyfunkionellen Verbindungen, Aminoharzen, Silanverbindungen, Diolverbindungen, Polyolverbindungen, Bisphenolverbindungen und Sulfiden vernetzt wurde.

3. Die perkutane pharmazeutische Zubereitung vom Absorptionstyp des Anspruchs 1, worin wenigstens eine der Haftkleberschicht (A) und der Haftkleberschicht (B) einen flüssigen Weichmacherbestandteil enthält.

4. Die perkutane pharmazeutische Zubereitung vom Absorptionstyp des Anspruchs 1, worin der Haftkleber der Haftkleberschicht (A) und der Haftkleber der Haftkleberschicht (B) dieselbe Zusammensetzung aufweisen.

5. Die perkutane pharmazeutische Zubereitung vom Absorptionstyp des Anspruchs 1, worin wenigstens je einer der Haftkleber der Haftkleberschicht (A) und der Haftkleber der Haftkleberschicht (B) ein Acrylcopolymer-Haftkleber sind.

6. Die perkutane pharmazeutische Zubereitung vom Absorptionstyp des Anspruchs 5, worin der Acrylcopolymer-Haftkleber der Haftkleberschicht (A) und der Haftkleberschicht (B) je ein Copolymer umfasst, das durch Copolymerisieren von 60 bis 98 Gew.-% von wenigstens einem Alkyl(meth)acrylat, in dem das Alkyl 4 bis 12 Kohlenstoffatome aufweist, mit 2 bis 40 Gew.-% wenigstens eines funktionellen Monomers erhalten wurde.

7. Die perkutane pharmazeutische Zubereitung vom Absorptionstyp des Anspruchs 6, worin das funktionelle Monomer ein Monomer ist, das einen oder mehreren Substituenten aufweist, die aus der Gruppe, bestehend aus einer Carboxylgruppe, einer Hydroxylgruppe, einer Sulfogruppe, einer Aminogruppe, einer Amidogruppe, einer Alkoxylgruppe, einer Cyanogruppe und einer Acyloxygruppe, ausgewählt sind.

8. Die perkutane pharmazeutische Zubereitung vom Absorptionstyp des Anspruchs 7, worin das funktionelle Monomer eines oder mehrere Monomere ist, ausgewählt aus der Gruppe, bestehend aus (Meth)acrylsäure, 2-Hydroxyethyl(meth)acrylat, Styrolsulfonsäure, (Meth)acrylamid, Vinylpyrrolidon, 2-Aminoethyl(meth)acrylat, Acrylonitril, 2-Methoxyethyl(meth)acrylat und Vinylacetat.

9. Ein Verfahren zum Herstellen einer pharmazeutischen Zubereitung vom perkutanen Absorptionstyp, das umfasst:
(1) einen Schritt des Formens einer Haftkleberschicht (A), umfassend einen Haftkleber, der durch physikalische Vernetzungsverfahren mit ultravioletter Strahlung oder Bestrahlung mit einem Elektronenstrahl vemetzt wurde, oder der mit einem Vernetzer, ausgewählt aus der Gruppe, bestehend aus einer metallorganischen Verbindung, einem Metallalkoholat oder einer Metallchelatverbindung vemetzt wurde, und ein basisches Arzneimittel auf einer Seite des Substrats; und
(2) einen Schritt des Vernetzens des Haftklebers mit einem anderen Vernetzer als einer metallorganischen Verbindung, einem Metallalkoholat und einer Metallchelatverbindung in Abwesenheit eines jeglichen basischen Arzneimittels, um einen vernetzten Haftkleber zu erhalten, und des Formens einer Haftkleberschicht (B), umfassend den vemetzten Haftkleber auf der Haftkleberschicht (A).

## Revendications

1. Préparation pharmaceutique de type à absorption percutanée qui comprend un substrat et, superposées sur un côté de celui-ci dans cet ordre,
une couche adhésive autocollante (A) comprenant un adhésif autocollant réticulé par des traitements de réticulation physiques avec une irradiation ultraviolette ou une irradiation par faisceau d'électrons, ou réticulé avec un agent de réticulation choisi dans un groupe constitué par un composé organométallique, un alcoolate de métal ou un composé chélate de métal, et un médicament basique, et
une couche adhésive autocollante (B) comprenant un adhésif autocollant réticulé avec un agent de réticulation autre qu'un composé organométallique, qu'un alcoolate de métal et qu'un composé chélate de métal.

2. Préparation pharmaceutique de type à absorption percutanée selon la revendication 1, dans laquelle l'adhésif autocollant de la couche adhésive autocollante (B) est un adhésif autocollant réticulé par un ou plusieurs agents de réticulation choisis dans le groupe constitué par les composés poly(isocyanate), les peroxydes organiques, les dérivés de mélamine, les composés polyfonctionnels, les résines aminées, les composés de silane, les composés de diol, les composés de polyol, les composés de bisphénol et les sulfures.

3. Préparation pharmaceutique de type à absorption percutanée selon la revendication 1, dans laquelle au moins l'une de la couche adhésive autocollante (A) et de la couche adhésive autocollante (B) contient un ingrédient plastifiant liquide.

4. Préparation pharmaceutique de type à absorption percutanée selon la revendication 1, dans laquelle l'adhésif autocollant de la couche adhésive autocollante (A) et l'adhésif autocollant de la couche adhésive autocollante (B) ont la même composition.

5. Préparation pharmaceutique de type à absorption percutanée selon la revendication 1, dans laquelle au moins l'un de l'adhésif autocollant de la couche adhésive autocollante (A) et de l'adhésif autocollant de la couche adhésive autocollante (B) est un adhésif autocollant copolymère acrylique.

6. Préparation pharmaceutique de type à absorption percutanée selon la revendication 5, dans laquelle l'adhésif autocollant copolymère acrylique de chacune de la couche adhésive autocollante (A) ou de la couche adhésive autocollante (B) comprend un copolymère obtenu en copolymérisant de 60 à 98 % en poids d'au moins un (méth)acrylate d'alkyle dans lequel le groupe alkyle comporte 4 à 12 atomes de carbone avec de 2 à 40 % en poids d'au moins un monomère fonctionnel.

7. Préparation pharmaceutique de type à absorption percutanée selon la revendication 6, dans laquelle le monomère fonctionnel est un monomère comportant un ou plusieurs substituants choisis dans le groupe constitué par un groupe carboxyle, un groupe hydroxyle, un groupe sulfo, un groupe amino, un groupe amido, un groupe alcoxyle, un groupe cyano et un groupe acyloxy.

8. Préparation pharmaceutique de type à absorption percutanée selon la revendication 7, dans laquelle le monomère fonctionnel est un ou plusieurs monomères choisis dans le groupe constitué par l'acide (méth)acrylique, le (méth)acrylate de 2-hydroxyéthyle, l'acide styrène sulfonique, le (méth)acrylamide, la vinylpyrrolidone, le (méth)acrylate de 2-aminoéthyle, l'acrylonitrile, le (méth)acrylate de 2-méthoxyéthyle et l'acétate de vinyle.

9. Procédé de production d'une préparation pharmaceutique de type à absorption percutanée qui comprend :
(1) une étape consistant à former une couche adhésive autocollante (A) comprenant un adhésif autocollant réticulé par des traitements de réticulation physiques avec une irradiation ultraviolette ou une irradiation par faisceau d'électrons, ou réticulé avec un agent de réticulation choisi dans le groupe constitué par un composé organométallique, un alcoolate de métal ou un composé chélate de métal, et un médicament basique sur un côté d'un substrat ; et
(2) une étape consistant à réticuler un adhésif autocollant avec un agent de réticulation autre qu'un composé organométallique, qu'un alcoolate de métal et qu'un composé chélate de métal en l'absence de tout médicament basique pour obtenir un adhésif autocollant réticulé et former une couche adhésive autocollante (B) comprenant l'adhésif autocollant réticulé sur la couche adhésive autocollante (A).
